Europäisches Patentamt

(19) European Patent Office

Office européen des brevets

(11) Numéro de publication : **0 171 321**
**B1**

(12) **FASCICULE DE BREVET EUROPÉEN**

(51) Int. Cl.⁵ : **A 61 F   5/56**

(45) Date de publication du fascicule du brevet :
**14.02.90**

(21) Numéro de dépôt : **85401454.5**

(22) Date de dépôt : **16.07.85**

(54) Dispositif destiné à interrompre le ronflement d'un dormeur.

(30) Priorité : **27.07.84 FR 8412115**

(43) Date de publication de la demande :
**12.02.86 Bulletin 86/07**

(45) Mention de la délivrance du brevet :
**14.02.90 Bulletin 90/07**

(84) Etats contractants désignés :
**AT BE CH DE FR GB IT LI LU NL SE**

(56) Documents cités :
**EP-A- 0 145 160**
**DE-B- 1 151 347**
**FR-A- 1 291 387**
**US-A- 3 696 377**
**US-A- 3 998 209**
**US-A- 4 220 142**

(73) Titulaire : **Mequignon, Jean Claude**
**10 Rue Honoré de Balzac**
**F-24120 Terrasson la Villedieu (FR)**

(72) Inventeur : **Mequignon, Jean Claude**
**10 Rue Honoré de Balzac**
**F-24120 Terrasson la Villedieu (FR)**

(74) Mandataire : **Ecrepont, Robert**
**Cabinet Ecrepont 12 Place Simon Vollant**
**F-59800 Lille (FR)**

## Description

L'invention concerne un dispositif destiné à interrompre dès son émission, le ronflement d'un être humain en train de dormir.

Le ronflement d'une personne est connu pour être incommodant et désagréable à supporter, et néfaste pour la santé.

Il existe actuellement des dispositifs dont le but est de « déconditionner » un dormeur ayant des prédispositions au ronflement.

Ces dispositifs généralement réagissent dès l'émission d'un ronflement de façon à établir une relation entre son ronflement et un phénomène précis, le plus souvent désagréable ou contrariant pour lui.

Par exemple, certains dispositifs déclenchent une alarme de façon à réveiller le dormeur, éventuellement l'obliger à se lever, ou le soumettre à une succession de sensations visuelles, auditives, tactiles dont le but principal est de provoquer chez le sujet une réaction de contrariété.

On connaît notamment les dispositifs décrits dans les brevets US-A-3 998 209 et US-A-4 220 142.

Ces dispositifs permettent en outre au dormeur d'évaluer ses progrès, en particulier le second dispositif qui comprend des moyens de mémorisation sur plusieurs jours.

De tels dispositifs sont efficaces, mais présentent l'inconvénient majeur de perturber profondément le sommeil du dormeur et de ses proches, ce qui peut avoir des répercutions dans leur comportement en dehors du sommeil.

On connaît également d'après le brevet US-A-3 696 377 un dispositif, qui dès l'émission du premier ronflement du dormeur, lui fait entendre un message de faible niveau sonore mais assez long pour lui enseigner les réflexes nécessaires pour qu'il se passe de cette habitude.

S'il est moins gênant pour les proches, ce dispositif, toutefois, est moins efficace que les deux dispositifs précédents car il agit de manière indirecte sur le subconscient du dormeur et n'amène plus nécessairement le dormeur à suivre la méthode enseignée.

Un des buts de la présente invention est de proposer un dispositif qui réagit dès l'émission du premier ronflement d'un dormeur et qui est réellement efficace pour interrompre le ronflement, sans toutefois perturber le sommeil du dormeur, c'est à dire sans le réveiller.

Un autre but de la présente invention est de proposer un dispositif qui permet au dormeur de connaître le matin, en se réveillant, le nombre de fois qu'il a, par ses ronflements, déclenché le système.

D'autres buts et avantages de la présente invention apparaîtront au cours de la description qui va suivre.

Le dispositif destiné à interrompre dès son émission, le ronflement d'un sujet, comprend un moyen tel un microphone pour capter le bruit émis par le sujet, des moyens pour mise hors et en circuit ce moyen capteur, des moyens pour comparer l'intensité du bruit capté par le moyen capteur à un seuil d'alarme déterminé, des moyens qui, en cas de dépassement du seuil d'alarme émettent durant un temps bref déterminé un stimulus sonore.

Il est caractérisé par le fait que ces moyens sont réglables pour émettre un stimulus d'intensité suffisante pour qu'il soit perçu par le sujet mais insuffisante pour l'éveiller et par le fait que les moyens pour mise hors et en circuit du moyen capteur mettent par ailleurs hors circuit le moyen capteur durant un temps déterminé supérieur au temps d'émission du son avant de le remettre automatiquement en circuit.

L'avantage du dispositif est d'agir sans aucun trouble du sommeil et sans perturbation du repos nocturne, les progrès réalisés par l'instauration d'une éducation-réflexe douce tendant à la diminution du nombre et de la puissance des ronflements.

L'invention sera mieux comprise à l'aide de la description qui à suivre, faite à titre d'exemple non limitatif, en regard du dessin ci-annexé qui représente schématiquement :

figure 1 : un schéma bloc illustrant le dispositif de l'invention,

figure 2 : un diagramme de temps illustrant l'action du dispositif de l'invention.

Le présent dispositif de l'invention a pour but d'interrompre dès son émission, le ronflement d'un être humain en train de dormir.

Le dispositif agit sans réveiller le sujet, et il a été élaboré à partir de l'observation que l'ouïe, d'une part, est le sens qui, lors de l'endormissement, s'endort le dernier, et, d'autre part qu'au cours du sommeil d'un être humain, c'est le sens qui dort le moins profondément.

Ainsi, lors d'un ronflement, le dispositif émet un stimulus sonore, de fréquence, d'intensité et de durée déterminées qui sans le réveiller provoque subconsciemment chez le sujet un changement de comportement entraînant l'interruption instantanée de son ronflement.

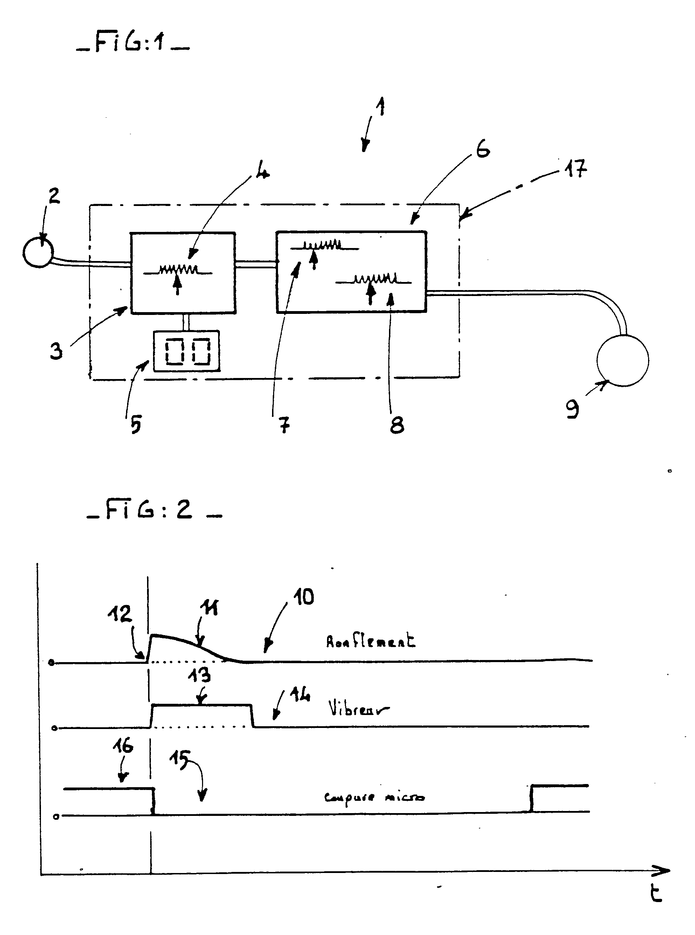

En se référant à la figure 1, le dispositif 1 de l'invention comprend d'une manière connue un microphone 2 qui est destiné à capter les bruits émis par le sujet.

A titre d'exemple, on a obtenu de bons résultats avec un microphone du type « Electret », sensibilisé à 50/60 dB environ.

Naturellement, tout autre microphone convient, et d'une manière plus générale, tout moyen pour capter les bruits émis par le sujet.

Le dispositif 1 comprend en outre des moyens schématisés en 3 pour comparer l'intensité du bruit capter par le microphone 2 à un seuil d'alarme déterminé.

D'une manière connue, ces moyens peuvent également présenter un dispositif de filtrage, éventuellement une temporisation, ou d'une manière générale tout moyen approprié pour éliminer, en dehors d'un ronflement, les autres bruits captés par le microphone 2.

Ces moyens de comparaison 3 comportent par ailleurs un réglage de sensibilité, schématisé en 4, qui permet de régler le seuil d'alarme auquel est comparé le bruit capté par le microphone 2. Eventuellement, des moyens de comptage schématisés en 5 comptabilisent le nombre de fois que l'intensité du bruit capté par le microphone dépasse le seuil d'alarme déterminé, pendant une période donnée, par exemple une nuit.

Ces moyens de comptage présentent par ailleurs une remise à zéro et éventuellement des moyens de mémorisation de la performance des jours précédents.

Il faut souligner que, dans le cas de l'invention, les moyens de comptage peuvent jouer un rôle important car, n'ayant pas été réveillé durant son sommeil, en s'éveillant le matin, le sujet n'a aucune idée du nombre de fois où il a déclenché l'appareil. Les moyens de comptage sont donc pour lui le moyen d'évaluer, sans aucun trouble du sommeil, et sans perturbation du repos nocturne, les progrès réalisés par l'instauration d'une éducation-réflexe douce tendant à la diminution du nombre et de la puissance des ronflements, contrairement aux autres dispositifs existants où le sujet a été réveillé un nombre de fois plus ou moins important pendant la nuit.

Selon une caractéristique de l'invention, le dispositif 1 comprend par ailleurs des moyens qui, en cas de dépassement du seuil d'alarme, émettent un stimulus durant un temps bref déterminé et à un niveau d'intensité suffisante pour qu'il soit perçu par le sujet mais insuffisante pour l'éveiller.

Ces moyens 6 entrent en action sous l'effet d'un signal reçu des moyens de comparaison 3 correspondant à un dépassement du seuil d'alarme.

Ils agissent durant un temps bref déterminé réglable au moyen d'une temporisation schématisée en 7, et émettent un stimulus dont l'intensité est réglable par les moyens schématisés en 8. On a obtenu de bons résultats avec un stimulus sonore émis par les moyens 6 dont la fréquence est de l'ordre de 2 000 Hz à plus ou moins dix pour cent, et notamment comprise entre 1 700 et 2 300 HZ.

Egalement, de bons résultats ont été obtenus par un stimulus sonore dont l'intensité est comprise entre 50 et 90 dB.

Un tel stimulus peut être obtenu par exemple par un vibreur schématisé en 9, un buzzer ou un ronfleur.

La durée d'émission du stimulus émis par ce vibreur 9 est réglée à une valeur de l'ordre de deux à trois secondes, et l'émission commence sensiblement dès le dépassement du seuil d'alarme.

Naturellement, ces chiffres n'ont qu'une valeur indicative.

En cas de dépassement du seuil d'alarme, et donc d'émission d'un stimulus par le vibreur 9, le microphone 2 est déconnecté automatiquement pendant une durée supérieure à l'émission du stimulus et au temps de réaction du sujet puis se retrouve automatiquement en circuit.

Le dispositif reprend alors sa veille ; éventuellement il émet de nouveau un stimulus par le vibreur 6 si le ronflement n'a pas cessé ou s'il reprend.

La durée de déconnexion peut être notamment de l'ordre de cinq à dix secondes.

La figure 2 illustre schématiquement le fonctionnement du dispositif.

La courbe supérieure 10 et notamment son créneau 11 correspondent au ronflement d'un sujet.

Dès l'émission de ce ronflement, schématisée en 12, ce vibreur 9 entre en action durant un temps déterminé schématisé par le créneau 13 de la courbe 14.

Egalement, le microphone est déconnecté durant un temps plus long que le temps d'émission du stimulus, ce qui est schématisé par le créneau 15 de la courbe 16.

Le stimulus émis par le vibreur dont l'intensité est suffisante pour qu'il soit perçu par le sujet mais insuffisante pour l'éveiller, provoque un changement de comportement subconscient du sujet interrompant son ronflement.

Si toutefois le ronflement continue, un nouveau stimulus est émis par le vibreur dès la remise en circuit du microphone 2.

Le dispositif de l'invention est réalisé avantageusement sous la forme d'un boîtier schématisé en 17 qui regroupe les moyens de comparaison 3 et les moyens d'émission 6.

Le microphone 2 peut être intégré à ce boîtier 17 ou bien il peut être indépendant et relié par un fil de liaison.

Le vibreur 9, quant à lui, est de préférence indépendant, et il est relié par un fil de longueur suffisante pour pouvoir être placé par exemple sous l'oreiller ou le traversin au niveau de la tête du sujet.

Il peut également, sans fil, être commandé par ondes radio ou tout autre moyen.

L'intensité du stimulus émis par le vibreur 9, réglable par les moyens 8 est réglée d'après l'acuité auditive et/ou la profondeur de sommeil du sujet.

Le boîtier 17 comprend par ailleurs des moyens d'alimentation appropriés, non représentés, qui peuvent être par exemple une alimentation électrique à partir du réseau alternatif, transformé ou un bloc de piles.

Naturellement, la présente description n'est donnée qu'à titre indicatif et l'on pourrait adopter d'autres mises en œuvre de l'invention sans pour autant sortir du cadre de celle-ci.

En particulier, à simple titre d'exemple, le vibreur pourrait être actionné par ondes radio commandées.

L'ensemble du dispositif, par les moyens existants ou à venir, pourrait tenir intégralement dans le conduit auditif et/ou le pavillon de l'oreille, etc...

## Revendications

1. Dispositif destiné à interrompre dès son émission, le ronflement d'un sujet, lequel dispositif qui comprend un moyen (2) tel un microphone pour capter le bruit émis par le sujet, des moyens pour mise hors et en circuit ce moyen capteur, des moyens (3) pour comparer l'intensité du bruit capté par le moyen capteur (2) à un seuil d'alarme déterminé, des moyens (6) qui, en cas de dépassement du seuil d'alarme émettent durant un temps bref déterminé un stimulus sonore, ce dispositif étant caractérisé par le fait que ces moyens (6) sont réglables pour émettre un stimulus d'intensité suffisante pour qu'il soit perçu par le sujet mais insuffisante pour l'éveiller et par le fait que les moyens pour mise hors et en circuit du moyen capteur mettent par ailleurs hors circuit le moyen capteur (2) durant un temps déterminé supérieur au temps d'émission du stimulus avant de le remettre automatiquement en circuit.

2. Dispositif selon la revendication 1 caractérisé par le fait que le stimulus est une onde sonore dont la fréquence est comprise entre 1 700 et 2 300 Hz.

3. Dispositif selon la revendication 1 caractérisé en ce que les dits moyens (6) comprennent un vibreur (9).

4. Dispositif selon la revendication 1 caractérisé par le fait que les dits moyens (6) comprennent des moyens (7) pour régler l'intensité du stimulus sonore émis entre 50 et 90 dB.

5. Dispositif selon la revendication 1 caractérisé par le fait que les dits moyens (6) comprennent des moyens (8) pour régler la durée d'émission du son émis entre deux et trois secondes après le dépassement du seuil d'alarme.

6. Dispositif selon la revendication 2 caractérisé par le fait que les dits moyens (6) comprennent des moyens pour mettre hors circuit le microphone (2) durant un temps réglable de cinq à dix secondes.

## Claims

1. Device intended for interrupting the snoring of a subject as soon as it is emitted, which device comprises a means (2) such as a microphone, for picking up the noise emitted by the subject, means for excluding this pickup means from the circuit and for re-inserting it, means (3) for comparing the intensity of the noise picked up by the pickup means (2) to a determined alarm threshold, means (6) which, in the event of the alarm threshold, being exceeded, emit a sound stimulus for a determined brief period of time, this device being characterised by the fact that these means (6) are regulatable so as to emit a stimulus which is of sufficient intensity for it to be perceived by the subject, but which is not sufficient to awaken the subject, and by the fact that the means for excluding the pickup means from the circuit and for re-inserting it furthermore exclude the pickup means (2) from the circuit for a determined period of time which is longer than the time of the emission of the stimulus, before automatically re-inserting it into the circuit.

2. Device according to claim 1, characterised by the fact that the stimulus is a sound wave whose frequency is comprised between 1 700 and 2 300 Hz.

3. Device according to claim 1, characterised in that the said means (6) comprise a vibrator (9).

4. Device according to claim 1, characterised by the fact that the said means (6) comprise means (7) for regulating the intensity of the sound stimulus emitted between 50 and 90 dB.

5. Device according to claim 1, characterised by the fact that the said means (6) comprise means (8) for regulating the duration of emission of the sound which is emitted between two and three seconds after the alarm threshold has been exceeded.

6. Device according to claim 2, characterised by the fact that the said means (6) comprise means for excluding the microphone (2) from the circuit for a regulatable period of time of five to ten seconds.

## Patentansprüche

1. Vorrichtung zur Unterbrechung des Schnarchens eines Schläfers, enthaltend eine Einrichtung (2), wie z. B. ein Mikrophon, zum Aufnehmen des vom Schläfer abgegebenen Geräuschs, Einrichtungen zu, Ein- und Ausschalten der Aufnahmeeinrichtung, Einrichtungen zu, Vergleich der Intensität des von der Aufnahmeeinrichtung aufgenommenen Geräuschs mit einer bestimmten Alarmschwelle, Einrichtungen (6), die beim Überschreiten der Alarmschwelle während eines kurzen Zeitraums einen lauten Stimulus abgibt, dadurch gekennzeichnet, daß die Einrichtung (6) so regelbar ist, daß ein Stimulus abgegeben wird, dessen Intensität ausreicht, daß es vom Schläfer aufgenommen wird, aber nicht ausreicht, um ihn aufzuwecken, und daß die Einrichtungen zum Ein- und Ausschalten der Aufnahmeeinrichtung die Aufnahmeeinrichtung (2) im übrigen während einer bestimmten Zeit abschalten, die länger ist als die Abgabezeit des Stimulus, bevor sie automatisch wieder einschalten.

2. Vorrichtung nach Anspruch 1, dadurch gekennzeichnet, daß der Stimulus eine Schallwelle mit einer Frequenz von 1 700 bis 2 300 Hz ist.

3. Vorrichtung nach Anspruch 1, dadurch gekennzeichnet, daß die Einrichtungen (6) einen Vibrator umfassen.

4. Vorrichtung nach Anspruch 1, dadurch gekennzeichnet, daß die Einrichtungen (6) Einrichtungen (7) umfassen zum Regeln der Intensität des abgegebenen Schallsignals von 50 bis 90 dB.

5. Vorrichtung nach Anspruch 1, dadurch gekennzeichnet, daß die Einrichtungen (6) Einrichtungen (8) umfassen zum Regeln der Schallabgabezeit von 2 bis 10 Sek. nach Überschreiten der Alarmschwelle.

6. Vorrichtung nach Anspruch 2, dadurch gekennzeichnet, daß die Einrichtungen (6) Einrichtungen umfassen zum Abschalten des Mikrophons (2) während einer regelbaren Zeit von 5 bis 10 Sek.

_FiG:1_

_FiG:2_

Ronflement

Vibreur

coupure micro

t